(19) Europäisches Patentamt
European Patent Office
Office européen des brevets

(11) EP 3 057 699 B1

(12) **FASCICULE DE BREVET EUROPEEN**

(45) Date de publication et mention de la délivrance du brevet:
**06.12.2017 Bulletin 2017/49**

(21) Numéro de dépôt: **14786844.2**

(22) Date de dépôt: **16.10.2014**

(51) Int Cl.:
***B01F 13/00*** (2006.01) ***B01J 13/04*** (2006.01)
***B01J 13/10*** (2006.01) ***B01F 3/08*** (2006.01)
*A61K 8/04* (2006.01) *A61K 8/37* (2006.01)
*A61Q 13/00* (2006.01) *A61Q 19/00* (2006.01)
*C11D 3/50* (2006.01)

(86) Numéro de dépôt international:
**PCT/EP2014/072176**

(87) Numéro de publication internationale:
**WO 2015/055748 (23.04.2015 Gazette 2015/16)**

(54) **PROCÉDÉ DE FORMATION D'UNE DISPERSION COMPRENANT DES GOUTTES, ET APPAREIL ASSOCIÉ**

VERFAHREN ZUR HERSTELLUNG EINER DISPERSION MIT TROPFEN UND ZUGEHÖRIGE VORRICHTUNG

METHOD FOR FORMING A DISPERSION COMPRISING DROPS, AND ASSOCIATED APPLIANCE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.10.2013 FR 1360098**

(43) Date de publication de la demande:
**24.08.2016 Bulletin 2016/34**

(73) Titulaire: **Capsum**
**13013 Marseille (FR)**

(72) Inventeurs:
- **GOUTAYER, Mathieu**
  **F-35400 Saint Malo (FR)**
- **PAFUMI, Yan, Eric**
  **F-13120 Gardanne (FR)**

(74) Mandataire: **Lavoix**
**2, place d'Estienne d'Orves**
**75441 Paris Cedex 09 (FR)**

(56) Documents cités:
**WO-A1-2012/089820 WO-A2-01/96009**
**WO-A2-2011/051033 FR-A1- 2 972 371**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 057 699 B1

## Description

La présente invention concerne un procédé de formation d'une dispersion contenant des gouttes, comprenant les étapes suivantes :

- écoulement, dans un conduit de circulation, de gouttes d'une première phase dans une deuxième phase sensiblement immiscible avec la première phase, chaque goutte comprenant un coeur formé de première phase et une écorce formée d'une couche de coacervat interposée entre la première phase et la deuxième phase ;
- récupération d'une dispersion comprenant des gouttes et de la deuxième phase dans un récipient.

Ce procédé est destiné par exemple à former une dispersion de type huile dans eau comprenant des gouttes stables de phase dispersée, de taille supérieure à 500 microns, et notamment comprise entre 500 microns et 2500 microns, de préférence entre 700 microns et 1500 microns.

La phase dispersée est destinée à contenir par exemple un produit cosmétique, un produit biologiquement actif, ou un produit comestible propre à être consommé.

La demande WO 2012/120043 décrit un procédé de fabrication du type précité. Ce procédé permet de fabriquer une dispersion stable de gouttes d'une première phase dans une deuxième phase sensiblement immiscible avec la première phase.

Chaque goutte comporte un coeur formé de première phase et une écorce très fine. L'écorce est formée d'une couche très fine de coacervat, interposée entre la première phase est la deuxième phase pour garantir la stabilité des gouttes.

À cet effet, dans le procédé, un premier fluide comprenant une première phase et un premier polymère précurseur du coacervat est injecté sous forme de gouttes dans un deuxième fluide destiné à former la deuxième phase.

Un deuxième polymère précurseur du coacervat est ensuite amené dans le deuxième fluide et diffuse jusqu'à l'interface avec les gouttes pour former la couche de coacervat.

Un tel procédé est particulièrement efficace pour former des gouttes de taille parfaitement contrôlée, de stabilité garantie, et présentant des propriétés optiques très satisfaisantes.

Toutefois, dans le cas des dispersions huile dans eau, la deuxième phase aqueuse doit être suffisamment fluide pour permettre une bonne émulsification au niveau du conduit d'injection de la première phase dans la deuxième phase.

Dans ces conditions, la dispersion obtenue à l'issue du procédé, lorsqu'elle est récupérée dans un récipient, présente une deuxième phase qui est rarement suspensive, avec une viscosité trop faible pour conférer au produit une texture agréable.

Par ailleurs, dans le cas où le polymère anionique formant une partie de la couche de coacervat est de type acide polyacrylique (carbomer ou dérivé), le pH de la deuxième phase est acide.

Pour pallier ce problème, la demande précitée propose d'ajouter des ingrédients a posteriori dans la deuxième phase, ou de remplacer la deuxième phase au moins partiellement par une autre composition aqueuse, afin d'augmenter la viscosité de la deuxième phase, voire de la gélifier.

Cette opération est délicate, puisque la membrane de coacervat entourant les gouttes d'huile est déformable et fragile. La manipulation du produit obtenu, une fois la phase aqueuse gélifiée, est en outre délicate. En effet, le cisaillement au niveau de l'interface eau/huile induit par l'écoulement, le mélange, ou le prélèvement sont susceptibles d'entraîner une déformation et/ou une rupture des gouttes d'huile, ce qui dégrade les propriétés visuelles du produit. Le document FR-A-2972371 décrit un procédé de fabrication selon le préambule de la revendication 1 et un appareil selon le préambule de la revendication 12. Un but de l'invention est donc de fournir un procédé simple d'obtention d'une dispersion contenant des gouttes en suspension stable dans d'une phase, tout en minimisant les manipulations à effectuer sur le produit.

À cet effet, l'invention a pour objet un procédé du type précité, caractérisé en ce que le procédé comporte l'étape suivante :

- injection d'une solution d'augmentation de la viscosité de la deuxième phase dans le conduit de circulation ou à la sortie du conduit de circulation, en amont du récipient.

Le procédé selon l'invention peut comprendre l'une ou plusieurs des caractéristiques suivantes, prise(s) isolément ou suivant toute combinaison techniquement possible :

- les gouttes et la deuxième phase s'écoulent suivant un axe local dans le conduit de circulation, l'injection de la solution d'augmentation de la viscosité s'effectuant sensiblement coaxialement avec l'axe local ;
- l'injection de la solution d'augmentation de la viscosité comporte l'amenée d'au moins une partie de la solution

d’augmentation de la viscosité au centre de l’écoulement des gouttes et de la deuxième phase ;

- l’injection de la solution d’augmentation de la viscosité comporte l’amenée d’au moins une partie de la solution d’augmentation de la viscosité à la périphérie de l’écoulement des gouttes et de la deuxième phase ;
- il comporte une réduction de la section transversale de l’écoulement des gouttes et de la deuxième phase, en aval de l’injection de la solution d’augmentation de la viscosité ;
- l’injection de la solution d’augmentation de la viscosité est effectuée à la sortie du conduit de circulation ;
- il comporte, en amont de l’étape de circulation, une étape de formation des gouttes dans le conduit de circulation ;
- l’étape de formation des gouttes comprend les sous étapes suivantes :

    ▪ fourniture d’un premier fluide comprenant la première phase et un premier polymère précurseur du coacervat contenu dans la première phase ;
    ▪ formation de gouttes de premier fluide dans un deuxième fluide destiné à former la deuxième phase, le deuxième fluide circulant avantageusement dans le conduit de circulation en amont de l’injection de la solution d’augmentation de la viscosité ;
    ▪ fourniture d’un deuxième polymère précurseur du coacervat dans le deuxième fluide ;

- il comporte, lors de l’étape d’injection, l’injection d’une première solution d’augmentation de la viscosité, adaptée pour augmenter la viscosité de la deuxième phase,
  le procédé comprenant après l’étape de récupération, les étapes suivantes :

    ▪ remise en circulation de la dispersion intermédiaire comprenant une deuxième phase de viscosité partiellement augmentée ;
    ▪ injection d’une deuxième solution d’augmentation de la viscosité dans la dispersion ;

- l’étape de remise en circulation comporte la circulation de la dispersion dans un conduit additionnel, puis la récupération de la dispersion dans un récipient à la sortie du conduit additionnel, l’injection de la deuxième solution d’augmentation de la viscosité étant effectuée dans le conduit additionnel, ou à la sortie du conduit additionnel, en amont du récipient ;
- la solution d’augmentation de la viscosité contient une base.

L’invention a également pour objet un appareil de formation d’une dispersion comprenant des gouttes, comprenant :

- un conduit de circulation contenant des gouttes de première phase dans une deuxième phase sensiblement immiscible avec la première phase, chaque goutte comprenant un coeur formé de première phase et une écorce formée d’une couche de coacervat interposée entre la première phase et la deuxième phase ;
- un récipient de récupération d’une dispersion comprenant des gouttes et la deuxième phase ;

caractérisé en ce que l’appareil comporte:

- un réservoir contenant une solution d’augmentation de la viscosité de la deuxième phase ;
- au moins un conduit d’injection de la solution d’augmentation de la viscosité, raccordé au réservoir, et débouchant dans le conduit de circulation ou à la sortie du conduit de circulation, en amont du récipient.

L’appareil selon l’invention peut comprendre l’une ou plusieurs des caractéristiques suivantes, prise(s) isolément ou suivant toute combinaison techniquement possible :

- le conduit de circulation s’étend suivant un axe local de circulation des gouttes et de la deuxième phase, le conduit d’injection débouchant coaxialement avec l’axe local ;
- il comporte un conduit périphérique d’injection d’au moins une partie de la solution d’augmentation de la viscosité, débouchant à la périphérie du conduit de circulation et/ou un conduit central d’injection d’au moins une partie de la solution d’augmentation de la viscosité, débouchant au centre du conduit de circulation ;
- il comporte un ensemble de formation de gouttes dans le conduit de circulation, l’ensemble de formation de gouttes comportant avantageusement :

    ▪ un conduit de fourniture d’un premier fluide comprenant la première phase et un premier polymère précurseur du coacervat contenu dans la première phase ;
    ▪ un conduit de formation de gouttes de premier fluide dans un deuxième fluide destiné à former la deuxième

phase, le deuxième fluide contenant ou recevant un deuxième polymère précurseur du coacervat.

L'invention sera mieux comprise à la lecture de la description qui va suivre, donnée uniquement à titre d'exemple, et faite en se référant aux dessins annexés, sur lesquels :

- la figure 1 est une vue de côté d'un récipient contenant une dispersion obtenue par un premier procédé selon l'invention ;
- la figure 2 est une vue en coupe d'une goutte de la dispersion selon l'invention ;
- la figure 3 est une vue schématique en coupe partielle d'un premier appareil de fabrication de la dispersion, pour la mise en oeuvre du premier procédé ;
- la figure 4 est une vue d'un détail de l'extrémité d'un conduit de circulation d'une variante d'appareil selon l'invention, où l'injection d'une solution d'augmentation de la viscosité est effectuée à l'extrémité du conduit de circulation ;
- la figure 5 est une vue de dessous de l'extrémité représentée sur la figure 4 ;
- la figure 6 est une vue analogue à la figure 3 d'une variante d'appareil de fabrication d'une dispersion, pour la mise en oeuvre d'un deuxième procédé selon l'invention ;
- la figure 7 est une vue en coupe analogue à la figure 3 d'un autre appareil selon l'invention.

Les figures 1 à 3 illustrent la mise en oeuvre d'un premier procédé de formation de gouttes selon l'invention.

En référence aux figures 1 et 2, ce premier procédé est destiné à former une dispersion 10 de gouttes 12 d'une première phase 14 dispersée dans une deuxième phase 16. La deuxième phase 16 est sensiblement immiscible avec la première phase 14.

Dans cet exemple, la première phase 14 ou phase dispersée est une phase huileuse. Elle contient notamment sous forme liquide un premier produit qui est choisi parmi un produit biologiquement actif, un produit cosmétique, ou un produit comestible propre à être consommé.

Lorsque le premier produit est un produit biologiquement actif, il est choisi avantageusement parmi les anticoagulants, les anti-thrombogéniques, les agents antimitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques.

En variante, la première phase 14 contient des agents réactifs tels que des protéines ou des réactifs destinés à former un bioréacteur, ou à former des cellules artificielles pour des implants.

Un produit cosmétique pouvant être contenu dans le coeur est par exemple cité dans la Directive 93/35/CEE du Conseil datée du 14 juin 1993. Ce produit est par exemple un parfum, une crème, une émulsion, une lotion, un gel et une huile pour la peau (mains, visage, pieds, etc.), un fond de teint (liquide, pâte) une préparation pour bains et douches (sels, mousses, huiles, gels, etc.), un produit de soins capillaires (teintures capillaires et décolorants), un produit de nettoyage (lotions, poudres, shampoings), un produit d'entretien pour la chevelure (lotions, crèmes, huiles), un produit de coiffage (lotions, laques, brillantines), un produit pour le rasage (savons, mousses, lotions, etc.), un produit destiné à être appliqué sur les lèvres , un produit solaire, un produit de bronzage sans soleil, un produit permettant de blanchir la peau, un produit antirides.

Les produits comestibles propres à être consommés par un être humain ou par un animal sont avantageusement des purées de légumes ou de fruits telles que la purée de mangue, de la purée de poire, de la purée de coco, de la crème d'oignons, de poireaux, de carottes, ou d'autres préparations pouvant mélanger plusieurs fruits ou légumes. En variante, il s'agit d'huiles telles qu'une huile alimentaire, du type huile d'olive, huile de soja, huile de grains de raisin, huile de tournesol, ou toute autre huile extraite des végétaux.

La deuxième phase 16 ou phase continue est avantageusement aqueuse. Lors de la formation des gouttes 12, la deuxième phase 16 est réalisée par exemple à partir d'une solution aqueuse ou hydroalcoolique.

La deuxième phase 16 comprend, dans une variante, des molécules d'intérêt cosmétique, tels que des actifs, des colorants, des stabilisants, des conservateurs, des agents modificateurs choisis parmi des agents de texture, de viscosité, de pH, de force osmotique ou des modificateurs d'indice de réfraction.

En variante, la première phase 14 est une phase aqueuse. La deuxième phase 16 est alors une phase huileuse.

L'huile constituant la phase huileuse est par exemple une huile siliconée, une huile minérale, une huile végétale, une huile synthétique ou un mélange de ces huiles.

Avantageusement, la dispersion 10 est sensiblement translucide.

Par sensiblement translucide, on entend que l'absorbance de la dispersion selon l'invention est généralement inférieure à 5%, de préférence inférieure 2%, préférentiellement inférieure à 1% pour au moins une longueur d'onde dans le spectre visible compris de 400 nanomètres à 1000 nanomètres, avantageusement sur toute longueur d'onde du spectre visible de 400 nanomètres à 1000 nanomètres.

L'intensité transmise à travers la dispersion 10 selon l'invention est divisée par un facteur d'au moins $10^3$ en

comparaison avec une émission concentrée classique.

Cette translucidité est mesurée en introduisant un échantillon de dispersion dans une cuve de 2 mm de trajet optique à une longueur d'onde comprise entre 400 nanomètres et 1000 nanomètres.

La première phase 14 et la deuxième phase 16 sont sensiblement immiscibles. Ainsi, la solubilité de la première phase 14 dans la deuxième phase 16 est inférieure avantageusement à 5 % en masse.

Le procédé selon l'invention permet d'obtenir une pluralité de gouttes 12 de première phase 14 tel qu'illustrées sur la figure 2.

Chaque goutte 12 comporte ainsi un coeur 17 constitué de première phase 14 et une écorce 18 de retenue et de stabilisation du coeur 17, l'écorce 18 étant formée par un coacervat entre un premier polymère précurseur et un deuxième polymère précurseur, comme décrit en détail plus bas.

Dans l'exemple représenté sur la figure 1, chaque goutte 12 est suspendue dans la deuxième phase 16 dans laquelle elle est dispersée.

Dans l'exemple représenté sur la figure 1, la viscosité de la deuxième phase 16 a été augmentée pour maintenir les gouttes 12 en suspension.

Initialement, lors de la formation des gouttes 12, la viscosité de la deuxième phase 16 est avantageusement inférieure à 20000 mPa.s, de préférence en dessous de 2500 mPa.s.

Une fois les gouttes 12 formées, la viscosité de la deuxième phase 16 est augmentée pour être supérieure à 3000 mPa.s, de préférence supérieure à 5000 mPa.s voire pour être totalement gélifiée.

Cette viscosité confère également à dispersion 10 une texture agréable au toucher.

L'augmentation de la viscosité, voire sa gélification, est obtenue par injection d'une solution d'augmentation de la viscosité, suivant le procédé qui sera décrit plus bas.

Dans le cas où la deuxième phase 16 est une phase aqueuse, la solution d'augmentation de la viscosité est par exemple une solution contenant une base, notamment un hydroxyde d'alcalin, tel que l'hydroxyde de sodium.

La viscosité est mesurée à température ambiante, par exemple T=25°C +/- 2°C et à pression ambiante, par exemple 1013 mbar, par la méthode suivante.

On utilise un viscosimètre de type Brookfield, typiquement un viscosimètre numérique Brookfield RVDV-E (couple de torsion du ressort de 7187,0 dyne-cm), qui est un viscosimètre rotationnel à vitesse imposée muni d'un mobile (désigné par le terme anglais « Spindle »). Une vitesse est imposée au mobile en rotation et la mesure du couple exercé sur le mobile permet de déterminer la viscosité en connaissant les paramètres de géométrie/forme du mobile utilisé.

On utilise par exemple un mobile de taille No. 04 (référence Brookfield: RV4). Le taux de cisaillement correspondant à la mesure de la viscosité est défini par le mobile utilisé et la vitesse de rotation de celui-ci.

La mesure de viscosité est effectuée sur 1 minute à température ambiante (T=25°C +/- 2°C). On place environ 150 g de solution dans un bêcher de 250 ml de volume, ayant un diamètre d'environ 7 cm de façon à ce que la hauteur du volume occupée par les 150 g de solution soit suffisante pour arriver à la jauge marquée sur le mobile. Ensuite, on démarre le viscosimètre sur une vitesse de 10 tours/min et on attend que la valeur affichée sur l'écran soit stable. Cette mesure donne la viscosité du fluide testé, telle que mentionnée dans le cadre de la présente invention.

En variante, dans le cas où la phase 16 est aqueuse, les gouttes huileuses 12 sont susceptibles de s'accumuler à la surface du récipient 33 qui reçoit la dispersion 10, si la viscosité de la deuxième phase 16 n'est pas modifiée.

Dans ce cas, les gouttes 12 sont disposées en appui les unes sur les autres. Par suite, la dispersion comprend au moins une région concentrée comportant des gouttes 12 et au moins une région dépourvue de gouttes 12 et comprenant exclusivement de la deuxième phase 16.

Dans l'exemple représenté sur les figures 1 et 2, le diamètre des gouttes 12 est supérieur à 500 μm, et est avantageusement inférieur à 3000 μm.

De manière avantageuse, le diamètre des gouttes 12 est compris entre 500 microns et 2500 microns. Les gouttes 12 sont visibles dans la phase aqueuse 16.

Dans un mode de réalisation, lorsque les gouttes 12 de la dispersion 10 possèdent une taille supérieure à 500 μm, les gouttes 12 présentent avantageusement une distribution de taille uniforme.

Plus précisément, selon ce mode, la phase dispersée est constituée d'une population de gouttes monodispersées 12 telles qu'elles possèdent un diamètre moyen $\overline{D}$ compris de 500 μm à 3 000 μm et un coefficient de variation Cv inférieur à 10%.

Dans le cadre de la présente description, on entend par « gouttes monodispersées » le fait que la population de gouttes de la dispersion selon l'invention possède une distribution de taille uniforme. Des gouttes monodispersées présentent une bonne monodispersité. A l'inverse, des gouttes présentant une mauvaise monodispersité sont dites « polydispersées ».

Le diamètre moyen $\overline{D}$ des gouttes est par exemple mesuré par analyse d'une photographie d'un lot constitué de N gouttes, par un logiciel de traitement d'image (Image J). Typiquement, selon cette méthode, le diamètre est mesuré en pixel, puis rapporté en μm, en fonction de la dimension du récipient contenant les gouttes 12 de la dispersion.

De préférence, la valeur de N est choisie supérieure ou égale à 30, de sorte que cette analyse reflète de

manière statistiquement significative la distribution de diamètres des gouttes de ladite émulsion.

On mesure le diamètre Di de chaque goutte, puis on obtient le diamètre moyen $\overline{D}$ en calculant la moyenne arithmétique de ces valeurs :

$$\overline{D} = \frac{1}{N}\sum_{i=1}^{N} D_i$$

A partir de ces valeurs $D_i$, on peut également obtenir l'écart-type $\sigma$ des diamètres des gouttes de la dispersion :

$$\sigma = \sqrt{\frac{\sum_{i=1}^{N}\left(D_i - \overline{D}\right)^2}{N}}$$

L'écart-type $\sigma$ d'une dispersion reflète la répartition des diamètres $D_i$ des gouttes de la dispersion autour du diamètre moyen $\overline{D}$.

En connaissant le diamètre moyen $\overline{D}$ et l'écart-type $\sigma$ d'une dispersion, on peut déterminer que l'on trouve 95,4% de la population de gouttes dans l'intervalle de diamètres $[\overline{D} - 2\sigma; \overline{D} + 2\sigma]$ et que l'on trouve 68,2% de la population dans l'intervalle $[\overline{D} - \sigma; \overline{D} + \sigma]$.

Pour caractériser la monodispersité de la dispersion selon ce mode de l'invention, on peut calculer le coefficient de variation :

$$C_v = \frac{\sigma}{\overline{D}}$$

Ce paramètre reflète la répartition des diamètres des gouttes en fonction du diamètre moyen de celles-ci.

Le coefficient de variation Cv des diamètres des gouttes de la dispersion 10 selon ce mode de l'invention est inférieur à 10%, de préférence inférieur à 5%.

Alternativement, la monodispersité peut être mise en évidence en plaçant un échantillon de dispersion 10 dans un flacon à section circulaire constante. Une agitation douce par rotation de un quart de tour sur une demi-seconde autour de l'axe de symétrie traversant le flacon, suivie d'un repos d'une demi-seconde est effectuée, avant de répéter l'opération en sens inverse, et ce quatre fois de suite.

Les gouttes 12 de la dispersion s'organisent sous une forme cristalline lorsqu'elles sont monodispersées. Ainsi, elles présentent un empilement suivant un motif se répétant suivant dans les trois dimensions. Il est alors possible d'observer, un empilement régulier qui indique une bonne monodispersité, un empilement irrégulier traduisant la polydispersité de la dispersion.

Lorsque la dispersion est laissée au repos, les gouttes 12 sont stables et n'adhèrent pas les unes aux autres, aucune coalescence n'est observée après 2 semaines à 40°C.

La dispersion 10 selon l'invention supporte une agitation sans cisaillement sans subir une coalescence importante des gouttes 12, ni une altération de sa monodispersité.

Pour tester cette propriété, un échantillon de la dispersion 10 selon l'invention est placé dans un récipient de 2 mL, puis celui-ci est placé dans une alvéole d'un plateau d'agitation (IKA vortex Genius 3). La taille d'une alvéole est environ 50% plus grande que celle du récipient contenant l'échantillon. Ainsi, lors de l'agitation le récipient heurte les parois de l'alvéole, ce qui créé un grand nombre de chocs. La vitesse d'agitation est d'environ 500 rpm. Le test est considéré comme réussi lorsque moins de 5% de gouttes en nombre ont subi une coalescence à l'issue d'une heure d'agitation sans cisaillement.

En plus des propriétés énoncées plus haut, de telles gouttes 12 sont non élastiques, et présentent un comportement granulaire lorsque la dispersion est agitée, de sorte qu'elles s'écoulent les unes sur les autres tels des objets solides et qu'elles adoptent un comportement particulaire en suspension.

En outre, la dispersion 10 selon l'invention peut présenter les propriétés suivantes.

Selon certains modes de réalisation avantageux, la dispersion 10 selon l'invention supporte une chute d'une hauteur d'un mètre sans subir une coalescence importante des gouttes 12, ni une altération de sa monodispersité.

Pour tester cette propriété, un échantillon de la dispersion 10 selon l'invention est placé dans un récipient de 2 mL, puis celui-ci est lâché en haut d'un tube en verre d'un mètre de hauteur servant de guide, sur un substrat solide,

et récupéré à son extrémité inférieure. L'opération est répétée trois fois. Le test est considéré comme réussi lorsque moins de 5% de gouttes 12 en nombre ont subi une coalescence à l'issue des trois chutes.

Les gouttes 12 sont sensiblement non élastiques. La non-élasticité des gouttes peut être caractérisée par un seuil de résistance à l'écoulement très faible, mesurée par exemple par la méthode suivante : un échantillon de dispersion 10 est placé dans un rhéomètre de type rhéometrics RFSII utilisant une géométrie cône-plan ayant un entrefer de 45 micromètres. Les cisaillements étudiés sont faibles, et sont notamment compris entre 1 $s^{-1}$ et 10 $s^{-1}$. Le module de cisaillement est mesuré pour des cisaillements croissants entre 1$s^{-1}$ et 10$s^{-1}$.

Ainsi, le module de cisaillement, sous un cisaillement faible, notamment égal à 2 $s^{-1}$, est inférieur à 200 Pa.s, et est notamment inférieur à 100 Pa.s.

Ce module de cisaillement est inférieur d'au moins 100 au module de cisaillement observé au même cisaillement, pour une dispersion classique stabilisée par des tensioactifs et présentant la même composition en huile et en eau.

En comparaison avec une émulsion classique (huile/eau) monodisperse concentrée à 80% (cf. Mason et al. J. Coll. Int. Sci. 179, 439-448 (1996)), une émulsion concentrée selon ce mode de réalisation présente une viscosité $\eta$ (Pa.s) inférieure d'un facteur 100, pour un cisaillement allant de 1 $s^{-1}$ à 12 $s^{-1}$.

Le coeur 17, même entouré de l'écorce 18, est sensiblement liquide ou peu gélifié. En variante, le coeur est gélifié.

Grâce au procédé selon l'invention, les gouttes 12 obtenues par ce procédé présentent une écorce 18 très fine, notamment d'épaisseur inférieure à 1 % du diamètre des gouttes 12.

L'épaisseur de l'écorce 18 est ainsi inférieure à 1 $\mu$m et est trop faible pour être mesurée par des méthodes optiques. Cette taille est généralement comprise entre 1 nm et 500 nm, de préférence inférieure à 100 nm, avantageusement inférieure à 50 nm, préférentiellement inférieure à 10 nm.

La mesure de l'épaisseur de l'écorce des gouttes 12 de l'invention peut être effectuée par la méthode de diffusion de neutrons aux petits angles (Small-Angle X-ray Scattering), telle que mise en oeuvre dans Sato et al. J. Chem. Phys. 111, 1393-1401 (2007).

Pour cela, les gouttes 12 sont produites en en utilisant de l'eau deutérée, puis sont lavées trois fois avec une huile deutérée, comme par exemple une huile deutérée de type hydrocarboné (octane, dodécane, hexadécane).

Après lavage, les gouttes 12 sont ensuite transférées dans la cellule de Neutrons afin de déterminer le spectre I(q) ; q étant le vecteur d'onde.

A partir de ce spectre, on applique les traitements analytiques classiques (REF) afin de déterminer l'épaisseur de l'écorce hydrogénée (non deutérée).

L'écorce 18 entourant les gouttes 12 de la dispersion selon l'invention est rigidifiée, ce qui confère une résistance supérieure aux gouttes 12 et diminue, voire empêche, leur coalescence.

Cette écorce 18 est typiquement formée par coacervation, c'est-à-dire par précipitation de polymères chargés de charges opposées. Au sein d'un coacervat, les liaisons liant les polymères chargés entre eux sont de type ionique, et sont généralement plus fortes que des liaisons de type électrostatique présentes au sein d'une membrane de type tensioactif.

L'écorce 18 est formée par coacervation d'au moins deux polymères chargés de polarité opposée (ou polyélectrolyte) et de préférence en présence d'un premier polymère, de type cationique, et d'un deuxième polymère, différent du premier polymère, de type anionique.

Dans le cadre de la présente description, on entend par « polymère de type anionique » un polymère comportant des fonctions chimiques de type anionique. On peut aussi parler de polyélectrolyte anionique.

Par « fonction chimique de type anionique », on entend une fonction chimique AH capable de céder un proton pour donner une fonction A. Selon les conditions du milieu dans lequel il se trouve, le polymère de type anionique comporte donc des fonctions chimiques sous forme AH, ou bien sous forme de sa base conjuguée $A^-$.

Comme exemple de fonctions chimiques de type anionique, on peut citer les fonctions acide carboxylique -COOH, éventuellement présentes sous forme d'anion carboxylate -COO-.

Comme exemple de polymère de type anionique, on peut citer tout polymère formé par la polymérisation de monomères dont au moins une partie porte des fonctions chimiques de type anionique, tel que des fonctions acide carboxylique. De tels monomères sont par exemple l'acide acrylique, l'acide maléique, ou tout monomère éthyléniquement insaturé comportant au moins une fonction acide carboxylique.

Parmi les exemples de polymère de type anionique appropriés à la mise en oeuvre de l'invention, on peut citer les copolymères d'acide acrylique ou d'acide maléique et d'autres monomères, tels que l'acrylamide, les acrylates d'alkyle, les acrylates d'alkyle en C5-C8, les acrylates d'alkyle en C10-C30, les méthacrylates d'alkyle en C12-C22, les méthacrylates méthoxypolyéthylèneglycol, les acrylates d'hydroxyester, les acrylates crosspolymères

Dans le cadre de la présente description, on entend par « polymère de type cationique » un polymère comportant des fonctions chimiques de type cationique. On peut aussi parler de polyélectrolyte cationique.

Par « fonction chimique de type cationique », on entend une fonction chimique B capable de capter un proton pour donner une fonction $BH^+$. Selon les conditions du milieu dans lequel il se trouve, le polymère de type cationique

comporte donc des fonctions chimiques sous forme B, ou bien sous forme $BH^+$, son acide conjugué.

Comme exemple de fonctions chimiques de type cationique, on peut citer les fonctions amine primaire, secondaire et tertiaire, éventuellement présentes sous forme de cations ammoniums.

Comme exemple de polymère de type cationique, on peut citer tout polymère formé par la polymérisation de monomères dont au moins une partie porte des fonctions chimiques de type cationique, tel que des fonctions amine primaire, secondaire ou tertiaire.

De tels monomères sont par exemple l'aziridine, ou tout monomère éthyléniquement insaturé comportant au moins une fonction amine primaire, secondaire ou tertiaire.

Parmi les exemples de polymère de type cationique appropriés à la mise en oeuvre de l'invention, on peut citer l'amodiméthicone, dérivé d'un polymère silicone (polydiméthylsiloxane, aussi appelé diméthicone), modifié par des fonctions amine primaire et amine secondaire :

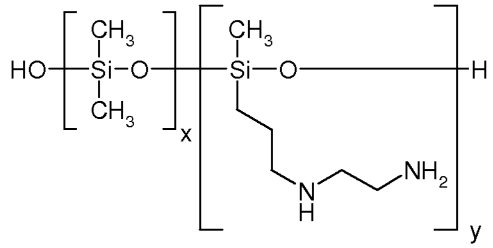

Amodiméthicone

On peut également citer des dérivés de l'amodiméthicone, comme par exemple des copolymères de l'amodiméthicone, l'aminopropyl diméthicone, et plus généralement des polymères silicones comportant des fonctions amines.

On peut citer le copolymère de bis-isobutyl PEG-14/amodiméthicone, le Bis (C13-15 Alkoxy) PG-Amodimethicone, le Bis-Cetearyl Amodimethicone et le bis-hydroxy/méthoxy amodiméthicone

On peut également citer les polymères de type polysaccharide comprenant des fonctions amine, tel que le chitosan ou les dérivés de gomme guar (chlorure d'hydroxypropyltrimonium guar).

On peut également citer les polymères de type polypeptide comprenant des fonctions amine, tel que la polylysine.

On peut également citer les polymères de type polyéthylèneimine comprenant des fonctions amine, tel que la polyéthylèneimine linéaire ou branchée.

La coacervation a généralement lieu en présence d'un premier polymère de type anionique et d'un deuxième polymère de type cationique, qui jouent le rôle d'agents de rigidification de la membrane.

La formation du coacervat entre ces deux polymères est généralement provoquée par une modification des conditions du milieu réactionnel (température, pH, concentration en réactifs, etc.). La réaction de coacervation résulte de la neutralisation de ces deux polymères chargés de polarités opposées et permet la formation d'une structure membranaire par interactions électrostatiques entre le premier et le deuxième polymère. La membrane ainsi formée autour de chaque goutte 12 forme une écorce 18 qui encapsule totalement le coeur 17 et isole la première phase 14 de la deuxième phase 16.

Comme on le verra plus bas dans la description du procédé selon l'invention, le premier polymère est contenu initialement dans l'une de la première phase 14 et de la deuxième phase 16, le deuxième polymère étant contenu initialement, avant la formation des gouttes 12, dans l'autre de la première phase 14 et de la deuxième phase 16. Les deux polymères migrent ensuite à l'interface lors de la formation des gouttes où ils forment l'écorce 18 par coacervation.

Une dispersion avantageuse est telle que chaque goutte 12 comprend, en masse par rapport à la masse de ladite goutte :

- de 0,05% à 10% d'un polymère P1 de type anionique et hydrophile, et
- de 0,05% à 10% d'un polymère P2 de type cationique et lipophile.

De préférence, chaque goutte 12 comprend, en masse par rapport à la masse de ladite goutte, de 0,1% à 5% d'un polymère P1 de type anionique et hydrophile.

De préférence, chaque goutte 12 comprend, en masse par rapport à la masse de ladite goutte, de 0,1% à 5% d'un polymère P2 de type cationique et lipophile.

Une dispersion avantageuse selon cette variante est telle que chaque goutte comprend un polymère P1, de type anionique et hydrophile, et un polymère P2, de type cationique et lipophile, dans un rapport massique P1 :P2 compris entre 1:10 et 10:1.

Le premier procédé selon l'invention est mis en oeuvre à l'aide d'une méthode microfluidique, dans un appareil 30 illustré par la figure 3.

Cet appareil 30 comporte une buse 32 de formation des gouttes 12, un étage 31 d'augmentation de la viscosité des gouttes 12 et un réceptacle 33 de réception des gouttes 12 formées.

La buse de formation 32 comporte un conduit interne 34 d'amenée d'un fluide interne 36 comprenant la première phase 14, et avantageusement, un conduit intermédiaire 37 d'amenée d'un fluide intermédiaire 39 destiné à former un écran temporaire.

La buse de formation 32 comporte en outre un conduit externe de circulation 38, disposé autour du conduit interne 34 et/ou du conduit intermédiaire 37 pour amener et faire circuler un fluide externe 40 formant au moins une partie de la deuxième phase 16.

L'appareil 30 comporte de plus des moyens 46 d'amenée de fluide interne 36 dans le conduit interne 34, des moyens 47 d'amenée de fluide intermédiaire 39 dans le conduit intermédiaire 37, et des moyens 48 d'amenée de fluide externe 40 dans l'espace annulaire délimité entre le conduit interne 34 et le conduit externe 38.

Dans l'exemple représenté sur la figure 3, le diamètre maximal des conduits 34, 37 et 38 est inférieur à 3 mm pour préserver le caractère microfluidique du procédé.

Le conduit interne 34 est avantageusement disposé coaxialement dans le conduit externe 38. Il est raccordé en amont aux moyens d'amenée 46. Il débouche en aval par une ouverture aval 52 disposée dans le conduit externe 38, en retrait par rapport à l'ouverture aval 54 définie par le conduit intermédiaire 37, au-dessus de cette ouverture 54.

Ainsi, la distance séparant l'ouverture aval 52 du conduit interne 34 et l'ouverture aval 54 du conduit intermédiaire 37 est de préférence supérieure à 1 fois le diamètre du conduit intermédiaire 37.

Le conduit intermédiaire 37 s'étend autour du conduit interne 34. Il délimite avec le conduit interne 34 un espace annulaire raccordé en amont aux moyens d'amenée 47. Le conduit intermédiaire 37 débouche par l'ouverture aval 54.

Le conduit externe 38 délimite avec le conduit intermédiaire 37 et/ou le conduit interne 34 un espace annulaire raccordé en amont aux moyens d'amenée 48.

Le conduit externe 38 présente une ouverture aval 55 qui est située au-dessus et à l'écart du récipient 33. Elle débouche dans l'étage d'augmentation de la viscosité 31.

Les moyens d'amenée 46, 47 et 48 comportent chacun par exemple un pousse seringue, une pompe péristaltique ou un autre système générateur de pression contrôlant le débit, comme par exemple un pot à pression couplé d'un débitmètre et d'un système de régulation du débit.

Chacun des moyens d'amenée 46, 47 et 48 est propre à convoyer un fluide respectif 36, 39, 40 à un débit contrôlé et réglable.

Selon l'invention, l'étage 31 comporte au moins un conduit 60 d'injection d'une solution 62 d'augmentation de la viscosité, et des moyens d'amenée 64 de la solution 62 dans le conduit 60.

Dans l'exemple représenté sur la figure 3, l'étage 31 comporte un conduit 60 périphérique d'injection d'au moins une partie de la solution d'augmentation de la viscosité 62.

Le conduit périphérique 60 s'étend à la périphérie du conduit externe de circulation 38, coaxialement avec l'axe local du conduit externe 38. L'ouverture aval 55 du conduit externe 38 s'étend dans le conduit périphérique 60.

Le conduit périphérique 60 définit, en aval de l'ouverture aval 55, une ouverture de distribution 66 qui débouche dans le récipient 33 ou au-dessus de celui-ci.

Le conduit périphérique 60 délimite, avec le conduit externe 38, un espace annulaire qui débouche en amont de l'ouverture de distribution 66 dans l'exemple représenté sur la figure 3.

Ainsi, le conduit périphérique 60 est configuré pour permettre l'injection de la solution d'augmentation de la viscosité 62 coaxialement avec l'axe de circulation de la dispersion contenant des gouttes 12 et la deuxième phase 16, juste à la sortie du conduit externe de circulation 38.

Dans cet exemple, le conduit périphérique 60 est propre à recueillir les gouttes 12 et la deuxième phase 16 dans laquelle la solution 62 d'augmentation de la viscosité a été introduite, et à les convoyer jusqu'à l'ouverture de distribution 66.

Les moyens 64 d'amenée comportent un réservoir 68 contenant la solution d'augmentation de la viscosité 62, et une unité de convoyage (non représentée).

L'unité de convoyage comporte par exemple un pousse seringue, une pompe péristaltique ou un autre système générateur de pression contrôlant le débit, comme par exemple un pot à pression couplé d'un débitmètre et d'un système de régulation du débit.

Le récipient 33 est disposé au-dessous de l'ouverture de distribution 66.

En variante, il contient un volume 70 de liquide destiné à former une partie de la deuxième phase 16, avantageusement un volume de fluide externe 40.

La surface supérieure du volume 70 de fluide est située axialement à l'écart de l'ouverture de distribution 66, prise le long de l'axe A-A' du conduit 60, de sorte que les gouttes 12 dispersées dans la deuxième phase 14 chutent sous l'effet de leur poids à travers un volume d'air entre l'ouverture de distribution 66 et la surface supérieure du volume

70 de liquide.

Dans une variante (non représentée), l'ouverture aval 54 est plongée dans le volume de liquide 70.

Dans l'exemple représenté sur la figure 3, le dispositif 30 a été illustré avec une seule buse 32, associé à un seul étage 31 d'augmentation de la viscosité.

Dans une variante avantageuse, illustrée en haut de la figure 6, le système 30 comporte une pluralité de buses 32, toutes reliées en aval à un étage commun 31 augmentation de la viscosité, les buses 32 étant disposées en parallèle au-dessus d'un récipient 33. Les buses 32 sont déportées latéralement par rapport à l'étage 31 d'augmentation de la viscosité. Un circuit de collecte permet de recueillir les gouttes 12 dans le liquide 40 en sortie de chaque buse 32 pour les rassembler et les introduire dans l'étage 31.

Un premier procédé selon l'invention, mis en oeuvre dans l'installation de la figure 3, va maintenant être décrit.

Initialement, le fluide interne 36 est préparé en mélangeant la première phase 14 destinée à former le coeur 17 de la goutte 12 et un premier polymère précurseur de la coacervation.

Dans cet exemple, le fluide interne 36 est avantageusement huileux. La première phase 14 contient au moins une huile, et un composé à disperser tel qu'un parfum. Le polymère précurseur est lipophile, et avantageusement cationique.

Le fluide intermédiaire 39 est également préparé. Ce fluide intermédiaire 39 est avantageusement huileux. Il est par exemple préparé à base de la même huile que celle contenue dans la première phase 14.

Le fluide externe 38 est aussi constitué. Dans cet exemple, le fluide externe 38 est aqueux. Il contient un deuxième polymère précurseur de la coacervation, ici un polymère hydrosoluble, par exemple de type anionique.

Avantageusement, des actifs, des colorants, des stabilisants, des conservateurs, des agents modificateurs choisis parmi des agents de texture, de viscosité, de pH, de force osmotique ou/et des modificateurs d'indice de réfraction sont ajoutés dans le fluide externe 38.

Une solution d'augmentation de la viscosité 62 est aussi préparée. Cette solution est avantageusement aqueuse. Elle comporte une base, notamment un hydroxyde d'alcalin, tels que l'hydroxyde de sodium.

Puis, le fluide interne 36, le fluide intermédiaire 37, et le fluide externe 40, sont disposés respectivement dans les moyens d'amenée respectifs 46, 47 et 48.

La solution d'augmentation de la viscosité 62 est disposée dans les moyens d'amenée 64.

Un liquide 70, formé d'un solvant aqueux de nature analogue à celle du fluide externe 40, est éventuellement introduit dans le récipient 33.

Ensuite, les moyens d'amenée 46, 47, 48 et 64 sont activés.

Le flux de fluide interne 36 circulant dans le conduit interne 34 entre coaxialement dans le conduit intermédiaire 37 au niveau de l'ouverture aval 52 du conduit interne 34.

Le fluide interne 36 est alors entouré par du fluide intermédiaire 39 dans le conduit intermédiaire 37.

Au niveau de l'ouverture aval 54 du conduit intermédiaire 37, des gouttes 12 de fluide interne 36, entourées par une pellicule de fluide intermédiaire 39, se forment dans le fluide externe 40.

Les gouttes 12 circulent alors dans le fluide externe 40 vers l'ouverture aval 55.

Le premier polymère présent dans le fluide interne 36 migre à l'interface entre les gouttes 12 et le fluide externe 40, par diffusion progressive dans la pellicule de fluide intermédiaire 39.

De même, le deuxième polymère présent dans le fluide externe 40 migre à l'interface entre le fluide externe 40 et chaque goutte 12.

La coacervation entre le premier polymère et le deuxième polymère se produit pour former l'écorce 18.

Le premier polymère et le deuxième polymère n'étant pas présents initialement dans la même phase, et une pellicule de fluide intermédiaire 39 dépourvu de polymères précurseurs étant présente initialement à la surface des gouttes 12, le risque qu'ils réagissent prématurément, notamment avant la formation des gouttes 12 de fluide interne 36 dans le fluide externe 40, est très limité. Ceci garantit que l'écorce 18 formée à l'interface entre la phase interne 14 et la phase externe 16 est complète, très mince, et n'engendre pas de gélification totale du coeur 17.

Les gouttes 12 ainsi formées sont donc très stables, peu ou pas élastiques et n'ont pas tendance à coalescer les unes sur les autres.

Puis, les gouttes 12 stabilisées dans le fluide externe 40, arrivent dans l'étage 31 d'augmentation de la viscosité.

La solution d'augmentation de la viscosité 62 est alors injectée coaxialement au flux de gouttes 12 dans le fluide externe 40, à la périphérie du fluide externe 40. La solution 62 diffuse dans le fluide externe 40 lors de son transport à travers la partie aval du conduit périphérique 60.

La viscosité du fluide externe 40 augmente alors significativement, pour atteindre une valeur supérieure à 3000 mPa.s, de préférence supérieure à 5000 mPa.s. Avantageusement, le fluide externe 40 est gélifié.

De même, le pH du fluide externe 40 est neutralisé.

L'augmentation de la viscosité et la neutralisation éventuelle se produisent au voisinage de l'ouverture de distribution 66, juste avant l'introduction de la dispersion 10 dans le récipient 33.

Au moins une goutte 12 est ensuite reçue dans une goutte extérieure 72 de fluide externe 40 qui est formée

à la sortie du conduit périphérique 60, au niveau de l'ouverture de distribution 66.

La goutte extérieure 72 tombe dans le récipient 33 à travers un volume d'air et les gouttes 12 de première phase 14 restent suspendues dans la deuxième phase 16 formée par le fluide externe 40 et par le liquide 70 lorsqu'un tel liquide est présent dans le récipient 33.

Dans une variante, le fluide externe 40 forme un jet à la sortie du conduit périphérique 60 et est collecté sans qu'il se fragmente. De préférence, le jet s'affine entre la sortie du conduit périphérique 60 et le récipient 33, afin de réduire le temps de diffusion de l'agent d'augmentation de la viscosité.

La neutralisation, et l'augmentation de la viscosité de la deuxième phase 16 sont donc conduites de manière peu invasive, et directement en ligne avec la fabrication des gouttes 12.

Ceci garantit l'utilisation d'une phase aqueuse suffisamment fluide pour autoriser une formation adéquate de gouttes 12 au niveau de la buse 30. Néanmoins, le produit final peut comprendre une viscosité satisfaisante pour lui conférer une texture agréable, sans nuire à la stabilité des gouttes 12 formées, et à moindre coût.

Le procédé selon l'invention est donc particulièrement efficace pour former des gouttes 12 stables, de dimensions supérieures à 500 μm, en suspension stable dans une phase 16, sans l'utilisation de tensio-actifs et de manière particulièrement contrôlée.

Le procédé selon l'invention limite le cisaillement, puisque la deuxième phase 16 continue contenant les gouttes 12 reste fluide jusqu'au dernier moment. Aucune force n'est engendrée pour déformer ou fragmenter les gouttes 12 lorsque la viscosité de la phase continue est augmentée.

Le crémage est également réduit. Le temps de diffusion de la solution 62 d'augmentation de la viscosité dans la phase continue 16 est très faible, compte tenu de la faible épaisseur à traverser. La phase continue 16 acquiert presque immédiatement un caractère suspensif lors de sa collecte dans le récipient 33.

Dans la variante représentée sur les figures 4 et 5, le conduit périphérique 60 débouche juste à la sortie du conduit externe 38. Le bord aval du conduit périphérique 60 est situé au même niveau horizontal que le bord aval du conduit externe 38.

L'ouverture de distribution 66 est alors située au même niveau horizontal que l'ouverture aval 55 du conduit externe de circulation 38.

Par ailleurs, l'étage 31 comporte un conduit central 80 d'injection d'au moins une partie de la solution 62 d'augmentation de la viscosité, qui s'étend au centre du conduit externe de circulation 38.

Dans cet exemple, le conduit central 80 débouche juste à la sortie du conduit externe 38. Son bord aval est situé au même niveau horizontal que le bord aval du conduit externe 38.

L'ouverture de distribution 82 du conduit central 80 est donc située au même niveau horizontal que l'ouverture aval 55 du conduit externe de circulation 38 et que l'ouverture de distribution 66 du conduit périphérique 60.

Cette conformation réduit l'épaisseur de l'écoulement comprenant les gouttes 12, le fluide externe 40, et la solution d'augmentation de la viscosité 62, puisque cet écoulement s'amincit par gravité en pénétrant dans le volume d'air situé à la sortie des conduits 38, 60, 80, comme illustré par la figure 5.

L'augmentation de la viscosité dans le fluide externe 40 est alors très homogène, puisque le temps de diffusion de la solution 62 diminue.

Dans une variante illustrée par la figure 6, le procédé de fabrication comporte une étape d'augmentation de la viscosité réalisée en deux sous-étapes.

Dans une première sous-étape 100, la quantité de base présente dans une première solution 62 d'augmentation de la viscosité, injectée juste après la formation des gouttes 12, et avant leur collecte dans le récipient 33, correspond à entre 1 % en masse et 90 % en masse, avantageusement entre 10 % en masse et 40 % en masse, de la quantité totale nécessaire pour réaliser l'augmentation de la viscosité en une étape.

La dispersion intermédiaire 102 obtenue à l'issue de la première sous-étape 100 présente alors une phase continue 16 qui suspend les gouttes 12, tout en restant manipulable.

La viscosité de la phase continue 16 dans la dispersion intermédiaire 102 est par exemple inférieure à 15000 mPa.s, et comprise entre 2500 mPa.s et 10000 mPa.s.

La dispersion intermédiaire 102 peut alors être mise en circulation, par gravité, par aspiration, ou par pression appliquée sur la dispersion 102.

Avantageusement, la solution d'augmentation de la viscosité 62 est injectée exclusivement par un conduit central 80 d'injection. De préférence, comme dans la configuration de la figure 4, le conduit central 80 débouche à l'extrémité du conduit externe de circulation 38, de sorte qu'un flux aminci se forme dans un volume d'air et tombe dans le récipient par gravité.

La chute libre et le faible impact à la réception homogénéisent la dispersion intermédiaire 102.

Puis, dans une deuxième sous-étape 104, la dispersion intermédiaire 102 est remise en circulation dans un conduit additionnel 106, muni à son extrémité d'au moins un conduit 60, 80 d'injection d'une deuxième solution d'augmentation de la viscosité 62, qui contient la quantité de base manquante.

De préférence, les conduits d'injection 60, 80 sont conformés comme dans l'appareil 30 de la figure 4, avec

un conduit périphérique 60 et un conduit central 80.

La dispersion finale 10 est alors récupérée dans le récipient 33.

Des exemples particuliers de mise en oeuvre du procédé selon l'invention pour obtenir des dispersions 10 vont maintenant être décrits.

Exemple 1

Une dispersion 10 de gouttes 12 de parfum, suspendues dans un gel aqueux est produite à l'aide d'un appareil 30 tel que schématisé dans la figure 3, en utilisant les fluides décrits ci-dessous, selon les conditions indiquées dans le Tableau 5.

Tableau 1 : Composition du fluide interne 36

| Nom | Nom INCI | % Masse |
|---|---|---|
| Lanol 99 | Isononyl Isononanoate | 0,5000 |
| Parfum | Parfum | 99,000 |
| KF 8004 | Amodimethicone | 0,5000 |
| Total | | 100,00 |

Tableau 2 : Composition du fluide intermédiaire 39

| Nom | Nom INCI | % Masse |
|---|---|---|
| Lanol 99 | Isononyl Isononanoate | 100,00 |
| Total | | 100,00 |

Tableau 3 : Composition du fluide externe 40

| Nom | Nom INCI | % Masse |
|---|---|---|
| Eau osmosée | Eau | 83,5918 |
| Glycerine codex (99%) | Glycérine | 6,2579 |
| Zemea propanediol | Propanediol | 6,2579 |
| Microcare PE | Phénoxyéthanol | 1,0013 |
| Microcare emollient PTG | Pentylène Glycol | 2,5032 |
| Rhodicare T | Gomme de Xanthane | 0,1252 |
| Tego carbomer 340 FD | Carbomer | 0,2503 |
| EDETA BD | Disodium EDTA | 0,0125 |
| Total | | 100,00 |

Tableau 4 : Composition de la solution 62 de gélification directe

| Nom | Nom INCI | % Masse |
|---|---|---|
| Eau osmosée | Eau | 99,6622 |
| NaOH | Hydroxyde de Sodium | 0,3378 |
| Total | | 100,00 |

Tableau 5 : Débits utilisés pour la préparation d'une dispersion de parfum avec une gélification directe

| | % w/w | ml/h/buse |
|---|---|---|
| Fluide externe 36 | 10,10% | 12,10 |
| Fluide intermédiaire 39 | 1,12% | 1,34 |
| Fluide externe 40 | 79,90% | 90,00 |
| Solution 62 | 8,88% | 10,00 |
| Total | 100 % | 113,45 |

A l'issue du procédé de fabrication, on récupère dans le flacon définitif 15 g d'une dispersion 10 de gouttes 12 de parfum, d'environ 1 mm de diamètre, ayant la composition décrite dans le Tableau 6.

Tableau 6 : composition finale de la dispersion 10 de gouttes 12 de parfum

| Nom | Nom INCI | % Masse |
|---|---|---|
| Eau osmosée | Eau | 75,64 |
| Lanol 99 | Isononyl Isononanoate | 1,17 |
| Parfum | Parfum | 10,00 |
| KF 8004 | Amodiméthicone | 0,05 |
| Glycerine codex (99%) | Glycérine | 5,00 |
| Zemea propanediol | Propanediol | 5,00 |
| Microcare PE | Phénoxyéthanol | 0,80 |
| Microcare emollient PTG | Pentylène glycol | 2,00 |
| Rhodicare T | Gomme de Xanthane | 0,10 |
| Tego carbomer 340 FD | Carbomer | 0,20 |
| EDETA BD | Disodium EDTA | 0,01 |
| NaOH | Hydroxyde de Sodium | 0,03 |
| Total | | 100,00 |

Exemple 2

Dans cet exemple on reprend les fluides 36, 39, 40 décrits ci-dessus respectivement dans les Tableaux 1 à 3. Toutefois, l'augmentation de la viscosité de la phase continue 16 s'effectue en deux sous étapes.

Une préparation de dispersion intermédiaire 102 de 2 kg est réalisée selon les conditions décrites dans le Tableau 8, à l'aide d'une première solution 62 illustrée dans le Tableau 7 ci-dessous.

Tableau 7 : composition de la première solution 62 de prégélification

| Nom | Nom INCI | % Masse |
|---|---|---|
| Eau osmosée | Eau | 97,5000 |
| NaOH | Hydroxyde de sodium | 2,5000 |
| Total | | 100,00 |

Tableau 8 : Débits utilisés pour la préparation d'une dispersion intermédiaire 102 de gouttes 12 de parfum

| | % w/w | ml/h /buse |
|---|---|---|
| Fluide interne 36 | 11,05% | 12,10 |

(suite)

| | % w/w | ml/h /buse |
|---|---|---|
| Fluide intermédiaire 39 | 1,23% | 1,34 |
| Fluide externe 40 | 87,43% | 90,00 |
| Première solution 62 | 0,29% | 0,30 |
| Total | 100 % | 103,75 |

La quantité de base ajoutée lors de cette sous étape correspond à 22,2 % en masse de la quantité finale désirée.

La dispersion intermédiaire 102 est ensuite amenée dans une trémie puis dans le conduit additionnel 106 afin de pouvoir être conditionnée dans le flacon définitif (15 ml).

Lors de cette sous étape, une deuxième solution 62 contenant le complément de base nécessaire pour atteindre le pH et la viscosité finale est ajoutée grâce à la solution de gélification finale décrite dans le Tableau 9 ci-dessous.

Pour obtenir les bonnes concentrations finales, la dispersion 102 et la solution 62 de gélification finale sont mises en contact dans les proportions massiques suivantes : respectivement 91,39 % et 8,61 %.

Ainsi, on obtient à l'issue du procédé de conditionnement, une dispersion 10 de gouttes 12 de parfum d'environ 1 mm de diamètre ayant la composition décrite dans le Tableau similaire à celle de l'exemple 1.

Tableau 9 : Composition de la solution de gélification finale

| Nom | Nom INCI | % w/w |
|---|---|---|
| Eau osmosée | Eau | 99,7291 |
| NaOH | Hydroxyde de Sodium | 0,2709 |
| Total | | 100,00 |

Dans une variante, représentée sur la figure 7, le conduit 60 d'injection d'une solution 62 d'augmentation de la viscosité comporte une couronne aval 210 de distribution de la solution 62 dans le conduit externe 38.

La couronne 210 est disposée au centre du conduit externe 38. Elle délimite, dans le conduit externe 38, un passage central 212 de circulation des gouttes 12, situé radialement à l'intérieur de la couronne aval 210, et un passage périphérique 214 de circulation des gouttes 12 situé radialement à l'extérieur de la couronne 210. Dans cet exemple, la couronne 210 est coaxiale avec l'axe A-A' local du conduit 38. La couronne aval 210 est en outre disposée en amont par rapport à l'ouverture aval 55 du conduit externe 38.

La couronne aval 210 comprend un tore 216 de répartition de la solution 62, et une pluralité de buses 218 de distribution, s'ouvrant dans le conduit externe 38 en aval de la couronne 210.

Le tore 216 s'étend sur une circonférence autour de l'axe A-A'. Il est raccordé à une partie amont 220 du conduit d'injection 60 par un pont de distribution 222.

Dans cet exemple, la couronne 210 comporte en outre une paroi inférieure 224 de support des buses 218 qui fait saillie vers la bas à partir du tore 216.

Chaque buse 218 est formée par un corps creux tubulaire, ici une aiguille, débouchant vers le haut dans le tore 216 et débouchant vers le bas dans le conduit externe 38. Les buses 218 s'étendent ici parallèlement à l'axe A-A'. Elles font saillie à partir de la paroi inférieure 224. Les buses 218 sont réparties angulairement autour de l'axe A-A'. Le nombre de buses 218 est par exemple compris entre 1 et 100.

Lors de l'injection de la solution 62 d'augmentation de la viscosité, la solution passe à travers la partie amont 220, à travers le pont 222 pour atteindre le tore 216. La solution se répartit dans le tore 216 autour de l'axe A-A' et passe dans les buses 218. Elle est donc injectée de manière régulière et homogène dans le conduit 38, entre les gouttes 12 circulant à travers le passage central 212 de la couronne 210 et les gouttes circulant à travers le passage périphérique 214 à l'extérieur de la couronne 210.

**Revendications**

1. Procédé de formation d'une dispersion (10) comprenant des gouttes (12), le procédé comportant les étapes suivantes :

- écoulement, dans un conduit de circulation (38), de gouttes (12) d'une première phase (14) dans une deuxième phase (16) sensiblement immiscible avec la première phase (14), chaque goutte comprenant un coeur (17) formé de première phase (14) et une écorce (18) formée d'une couche de coacervat interposée entre la première phase (14) et la deuxième phase (16) ;
- récupération d'une dispersion (10) comprenant des gouttes (12) et de la deuxième phase (16) dans un récipient (33) ;

**caractérisé en ce que** le procédé comporte les étapes suivantes :

- injection d'une solution (62) d'augmentation de la viscosité de la deuxième phase (16) dans le conduit de circulation (38) ou à la sortie du conduit de circulation (38), en amont du récipient (33).

**2.** Procédé selon la revendication 1, **caractérisé en ce que** les gouttes (12) et la deuxième phase (16) s'écoulent suivant un axe local dans le conduit de circulation (38), l'injection de la solution (62) d'augmentation de la viscosité s'effectuant sensiblement coaxialement avec l'axe local.

**3.** Procédé selon l'une quelconque des revendications précédentes, **caractérisé en ce que** l'injection de la solution (62) d'augmentation de la viscosité comporte l'amenée d'au moins une partie de la solution (62) d'augmentation de la viscosité au centre de l'écoulement des gouttes (12) et de la deuxième phase (16).

**4.** Procédé selon l'une quelconque des revendications précédentes, **caractérisé en ce que** l'injection de la solution (62) d'augmentation de la viscosité comporte l'amenée d'au moins une partie de la solution (62) d'augmentation de la viscosité à la périphérie de l'écoulement des gouttes (12) et de la deuxième phase (16).

**5.** Procédé selon l'une quelconque des revendications précédentes, **caractérisé en ce qu'**il comporte une réduction de la section transversale de l'écoulement des gouttes (12) et de la deuxième phase (16), en aval de l'injection de la solution (62) d'augmentation de la viscosité, l'injection de la solution (62) d'augmentation de la viscosité étant avantageusement effectuée à la sortie du conduit de circulation (38).

**6.** Procédé selon l'une quelconque des revendications précédentes, **caractérisé en ce qu'**il comporte, en amont de l'étape de circulation, une étape de formation des gouttes (12) dans le conduit de circulation (38).

**7.** Procédé selon la revendication 6, **caractérisé en ce que** l'étape de formation des gouttes (12) comprend les sous étapes suivantes :

- fourniture d'un premier fluide (36) comprenant la première phase (14) et un premier polymère précurseur du coacervat contenu dans la première phase (14) ;
- formation de gouttes (12) de premier fluide (36) dans un deuxième fluide (40) destiné à former la deuxième phase (16), le deuxième fluide (40) circulant avantageusement dans le conduit de circulation (38) en amont de l'injection de la solution (62) d'augmentation de la viscosité ;
- fourniture d'un deuxième polymère précurseur du coacervat dans le deuxième fluide (40).

**8.** Procédé selon l'une quelconque des revendications précédentes, **caractérisé en ce qu'**il comporte, lors de l'étape d'injection, l'injection d'une première solution (62) d'augmentation de la viscosité, adaptée pour augmenter la viscosité de la deuxième phase (16),
le procédé comprenant après l'étape de récupération, les étapes suivantes :

- remise en circulation de la dispersion intermédiaire (102) comprenant une deuxième phase (16) de viscosité partiellement augmentée ;
- injection d'une deuxième solution (62) d'augmentation de la viscosité dans la dispersion (102).

**9.** Procédé selon la revendication 8, **caractérisé en ce que** l'étape de remise en circulation comporte la circulation de la dispersion (10) dans un conduit additionnel (106), puis la récupération de la dispersion (10) dans un récipient (33) à la sortie du conduit additionnel (106), l'injection de la deuxième solution (62) d'augmentation de la viscosité étant effectuée dans le conduit additionnel (106), ou à la sortie du conduit additionnel (106), en amont du récipient (33).

**10.** Procédé selon l'une quelconque des revendications précédentes, **caractérisé en ce que** la solution (62) d'augmentation de la viscosité contient une base.

**11.** Procédé selon l'une quelconque des revendications précédentes, **caractérisé en ce que** l'injection de la solution (62) d'augmentation de la viscosité comporte l'amenée d'au moins une partie de la solution (62) d'augmentation de la viscosité dans une couronne (210) de distribution de la solution située au centre du conduit de circulation (38), les gouttes (12) s'écoulant dans un passage central (212) délimité par la couronne (210) et dans un passage périphérique (214) délimité entre la couronne et le conduit de circulation (38).

**12.** Appareil (30) de formation d'une dispersion (10) comprenant des gouttes (12), comprenant :

- un conduit de circulation (38) contenant des gouttes (12) de première phase (14) dans une deuxième phase (16) sensiblement immiscible avec la première phase (14), chaque goutte (12) comprenant un coeur (17) formé de première phase (14) et une écorce (18) formée d'une couche de coacervat interposée entre la première phase (14) et la deuxième phase (16) ;
- un récipient (33) de récupération d'une dispersion (10) comprenant des gouttes (12) et la deuxième phase (16) ;

**caractérisé en ce que** l'appareil (30) comporte:

- un réservoir (68) contenant une solution (62) d'augmentation de la viscosité de la deuxième phase (16) ;
- au moins un conduit (60 ; 80) d'injection de la solution (62) d'augmentation de la viscosité, raccordé au réservoir (68), et débouchant dans le conduit de circulation (38) ou à la sortie du conduit de circulation (38), en amont du récipient (33).

**13.** Appareil (30) selon la revendication 12, **caractérisé en ce que** le conduit de circulation (38) s'étend suivant un axe local de circulation des gouttes (12) et de la deuxième phase (16), le conduit d'injection (60 ; 80) débouchant coaxialement avec l'axe local.

**14.** Appareil (30) selon l'une quelconque des revendications 12 ou 13, **caractérisé en ce qu'**il comporte un conduit périphérique (60) d'injection d'au moins une partie de la solution (62) d'augmentation de la viscosité, débouchant à la périphérie du conduit de circulation (38) et/ou un conduit central (80) d'injection d'au moins une partie de la solution (62) d'augmentation de la viscosité, débouchant au centre du conduit de circulation (38).

**15.** Appareil (30) selon l'une quelconque des revendications 12 à 14, **caractérisée en ce qu'**il comporte un ensemble de formation de gouttes (12) dans le conduit de circulation (38), l'ensemble de formation de gouttes (12) comportant avantageusement :

- un conduit (34) de fourniture d'un premier fluide (36) comprenant la première phase (14) et un premier polymère précurseur du coacervat contenu dans la première phase (14) ;
- un conduit (37) de formation de gouttes (12) de premier fluide (36) dans un deuxième fluide (40) destiné à former la deuxième phase (16), le deuxième fluide (40) contenant ou recevant un deuxième polymère précurseur du coacervat.

## Patentansprüche

**1.** Verfahren zum Bilden einer Dispersion (10) mit Tropfen (12), wobei das Verfahren die folgenden Schritte umfasst:

- Bewirken einer Strömung von Tropfen (12) einer ersten Phase (14) in einer zweiten Phase (16), die mit der ersten Phase (14) im Wesentlichen nicht mischbar ist, in einer Zirkulationsleitung (38), wobei jeder Tropfen einen Kern (17), der aus der ersten Phase (14) gebildet ist, und eine Schale (18), die aus einer Koazervatschicht gebildet ist, die zwischen die erste Phase (14) und die zweite Phase (16) eingefügt ist, umfasst;
- Wiedergewinnen einer Dispersion (10) mit Tropfen (12) und der zweiten Phase (16) in einem Behälter (33);

**dadurch gekennzeichnet, dass** das Verfahren die folgenden Schritte umfasst:

- Einleiten einer Lösung (62) zur Erhöhung der Viskosität der zweiten Phase (16) in die Zirkulationsleitung (38) oder am Ausgang der Zirkulationsleitung (38) stromaufwärts des Behälters (33).

**2.** Verfahren nach Anspruch 1, **dadurch gekennzeichnet, dass** die Tropfen (12) und die zweite Phase (16) entlang einer lokalen Achse in der Zirkulationsleitung (38) strömen, wobei das Einleiten der Lösung (62) zur Erhöhung der

Viskosität im Wesentlichen koaxial mit der lokalen Achse erfolgt.

**3.** Verfahren nach einem der vorangehenden Ansprüche, **dadurch gekennzeichnet, dass** das Einleiten der Lösung (62) zur Erhöhung der Viskosität das Zuführen von mindestens einem Teil der Lösung (62) zur Erhöhung der Viskosität in der Mitte der Strömung der Tropfen (12) und der zweiten Phase (16) umfasst.

**4.** Verfahren nach einem der vorangehenden Ansprüche, **dadurch gekennzeichnet, dass** das Einleiten der Lösung (62) zur Erhöhung der Viskosität das Zuführen von mindestens einem Teil der Lösung (62) zur Erhöhung der Viskosität am Umfang der Strömung der Tropfen (12) und der zweiten Phase (16) umfasst.

**5.** Verfahren nach einem der vorangehenden Ansprüche, **dadurch gekennzeichnet, dass** es eine Verringerung des Querschnitts der Strömung der Tropfen (12) und der zweiten Phase (16) stromabwärts der Einleitung der Lösung (62) zur Erhöhung der Viskosität umfasst, wobei das Einleiten der Lösung (62) zur Erhöhung der Viskosität vorteilhafterweise am Ausgang der Zirkulationsleitung (38) durchgeführt wird.

**6.** Verfahren nach einem der vorangehenden Ansprüche, **dadurch gekennzeichnet, dass** es vor dem Zirkulationsschritt einen Schritt des Bildens der Tropfen (12) in der Zirkulationsleitung (38) umfasst.

**7.** Verfahren nach Anspruch 6, **dadurch gekennzeichnet, dass** der Schritt des Bildens der Tropfen (12) die folgenden Unterschritte umfasst:

- Liefern eines ersten Fluids (36) mit der ersten Phase (14) und einem ersten Vorläuferpolymer des Koazervats, das in der ersten Phase (14) enthalten ist;
- Bilden von Tropfen (12) des ersten Fluids (36) in einem zweiten Fluid (40), das die zweite Phase (16) bilden soll, wobei das zweite Fluid (40) vorteilhafterweise in der Zirkulationsleitung (38) stromaufwärts der Einleitung der Lösung (62) zur Erhöhung der Viskosität zirkuliert;
- Liefern eines zweiten Vorläuferpolymers des Koazervats in das zweite Fluid (40).

**8.** Verfahren nach einem der vorangehenden Ansprüche, **dadurch gekennzeichnet, dass** es im Einleitungsschritt das Einleiten einer ersten Lösung (62) zur Erhöhung der Viskosität umfasst, die dazu ausgelegt ist, die Viskosität der zweiten Phase (16) zu erhöhen,
wobei das Verfahren nach dem Wiedergewinnungsschritt die folgenden Schritte umfasst:

- erneutes Bewirken eines Zirkulierens der Zwischendispersion (102) mit einer zweiten Phase (16) mit teilweise erhöhter Viskosität;
- Einleiten einer zweiten Lösung (62) zur Erhöhung der Viskosität in die Dispersion (102).

**9.** Verfahren nach Anspruch 8, **dadurch gekennzeichnet, dass** der Schritt des erneuten Bewirkens des Zirkulierens das Bewirken der Zirkulation der Dispersion (10) in einer zusätzlichen Leitung (106) und dann das Wiedergewinnen der Dispersion (10) in einem Behälter (33) am Ausgang der zusätzlichen Leitung (106) umfasst, wobei das Einleiten der zweiten Lösung (62) zur Erhöhung der Viskosität in der zusätzlichen Leitung (106) oder am Ausgang der zusätzlichen Leitung (106) stromaufwärts des Behälters (33) durchgeführt wird.

**10.** Verfahren nach einem der vorangehenden Ansprüche, **dadurch gekennzeichnet, dass** die Lösung (62) zur Erhöhung der Viskosität eine Base enthält.

**11.** Verfahren nach einem der vorangehenden Ansprüche, **dadurch gekennzeichnet, dass** das Einleiten der Lösung (62) zur Erhöhung der Viskosität das Zuführen zumindest eines Teils der Lösung (62) zur Erhöhung der Viskosität in eine Ringleitung (210) zur Verteilung der Lösung umfasst, die in der Mitte der Zirkulationsleitung (38) angeordnet ist, wobei die Tropfen (12) in einem zentralen Durchgang (212), der durch die Ringleitung (210) begrenzt ist, und in einem Umfangsdurchgang (214), der zwischen der Ringleitung und der Zirkulationsleitung (38) begrenzt ist, strömen.

**12.** Gerät (30) zum Bilden einer Dispersion (10) mit Tropfen (12), das umfasst:

- eine Zirkulationsleitung (38), die Tropfen (12) einer ersten Phase (14) in einer zweiten Phase (16) enthält, die mit der ersten Phase (14) im Wesentlichen nicht mischbar ist, wobei jeder Tropfen (12) einen Kern (17), der aus der ersten Phase (14) gebildet ist, und eine Schale (18), die aus einer Koazervatschicht gebildet ist, die

zwischen die erste Phase (14) und die zweite Phase (16) eingefügt ist, umfasst;
- einen Behälter (33) zum Wiedergewinnen einer Dispersion (10) mit Tropfen (12) und der zweiten Phase (16);

**dadurch gekennzeichnet, dass** das Gerät (30) ferner umfasst:

- ein Reservoir (68), das eine Lösung (62) zur Erhöhung der Viskosität der zweiten Phase (16) enthält;
- mindestens eine Leitung (60; 80) zum Einleiten der Lösung (62) zur Erhöhung der Viskosität, die mit dem Reservoir (68) verbunden ist und in die Zirkulationsleitung (38) oder am Ausgang der Zirkulationsleitung (38) stromaufwärts des Behälters (33) mündet.

**13.** Gerät (30) nach Anspruch 12, **dadurch gekennzeichnet, dass** sich die Zirkulationsleitung (38) entlang einer lokalen Zirkulationsachse der Tropfen (12) und der zweiten Phase (16) erstreckt, wobei die Einleitungsleitung (60; 80) koaxial mit der lokalen Achse mündet.

**14.** Gerät (30) nach einem der Ansprüche 12 oder 13, **dadurch gekennzeichnet, dass** es eine Umfangsleitung (60) zum Einleiten mindestens eines Teils der Lösung (62) zur Erhöhung der Viskosität, die am Umfang der Zirkulationsleitung (38) mündet, und/oder eine zentrale Leitung (80) zum Einleiten mindestens eines Teils der Lösung (62) zur Erhöhung der Viskosität, die in der Mitte der Zirkulationsleitung (38) mündet, umfasst.

**15.** Gerät (30) nach einem der Ansprüche 12 bis 14, **dadurch gekennzeichnet, dass** es eine Einheit zum Bilden von Tropfen (12) in der Zirkulationsleitung (38) umfasst, wobei die Einheit zum Bilden von Tropfen (12) vorteilhafterweise umfasst:

- eine Leitung (34) zum Liefern eines ersten Fluids (36) mit der ersten Phase (14) und einem ersten Vorläuferpolymer des Koazervats, das in der ersten Phase (14) enthalten ist;
- eine Leitung (37) zum Bilden von Tropfen (12) des ersten Fluids (36) in einem zweiten Fluid (40), das die zweite Phase (16) bilden soll, wobei das zweite Fluid (40) ein zweites Vorläuferpolymer des Koazervats enthält oder empfängt.

## Claims

**1.** A method for forming a dispersion (10) comprising drops (12), the method including the following steps:

- flowing drops (12) of a first phase (14), through a circulation duct (38), into a second phase (16) that is substantially immiscible with the first phase (14), each drop comprising a core (17) formed from the first phase (14) and a shell (18) formed from a layer of coacervate inserted between the first phase (14) and the second phase (16);
- recovering a dispersion (10) containing drops (12) and second phase (16) in a container (33);

**characterized in that** the method includes the following steps:

- injecting a solution (62) for increasing the viscosity of the second phase (16) in the circulation duct (38) or at the outlet of the circulation duct (38), upstream from the container (33).

**2.** The method according to claim 1, **characterized in that** the drops (12) of the second phase (16) flow along a local axis in the circulation duct (38), injecting the solution (62) increasing the viscosity being done substantially coaxially with the local axis.

**3.** The method according to any one of the preceding claims, **characterized in that** injecting the solution (62) increasing the viscosity includes bringing at least part of the solution (62) increasing the viscosity to the center of the flow of the drops (12) and the second phase (16).

**4.** The method according to any one of the preceding claims, **characterized in that** injecting the solution (62) increasing the viscosity includes bringing at least part of the solution (62) increasing the viscosity to the periphery of the flow of the drops (12) and the second phase (16).

**5.** The method according to any one of the preceding claims, **characterized in that** it includes reducing the cross-section of the flow of drops (12) and the second phase (16), downstream from the injection of the solution (62) increasing the viscosity, the solution (62) increasing the viscosity being advantageously injected at the outlet of the circulation duct (38).

**6.** The method according to any one of the preceding claims, **characterized in that** it includes, upstream from the circulation step, a step for forming drops (12) in the circulation duct (38).

**7.** The method according to claim 6, **characterized in that** the step for forming drops (12) comprises the following sub-steps:

- providing a first fluid (36) comprising the first phase (14) and a first precursor polymer of the coacervate contained in the first phase (14);
- forming drops (12) of first fluid (36) in a second fluid (40) intended to form the second phase (16), the second fluid (40) advantageously circulating in the circulation duct (38) upstream from the injection of the solution (62) increasing the viscosity;
- providing a second precursor polymer of the coacervate in the second fluid (40).

**8.** The method according to any one of the preceding claims, **characterized in that** it includes, during the injection step, the injection of a first solution (62) increasing the viscosity, suitable for increasing the viscosity of the second phase (16),
the method comprising, after the recovery step, the following steps:

- returning the intermediate dispersion (102), comprising a second phase (16) with a partially increased viscosity, to circulation;
- injecting a second solution (62) increasing the viscosity into the dispersion (102).

**9.** The method according to claim 8, **characterized in that** the recirculation step includes the circulation of the dispersion (10) in an additional duct (106), then the recovery of the dispersion (10) in a container (33) at the outlet of the additional duct (106), the injection of the second solution (62) increasing the viscosity being done in the additional duct (106), or at the outlet of the additional duct (106), upstream from the container (33).

**10.** The method according to any one of the preceding claims, **characterized in that** the solution (62) for increasing the viscosity contains a base.

**11.** The method according to any one of the preceding claims, **characterized in that** the injection of the solution (62) increasing the viscosity includes bringing at least part of the solution (62) increasing the viscosity to the center of the circulation duct (38), the drops (12) flowing in a central passage (212) defined by the crown (210) and in a peripheral passage (214) defined between the crown and the circulation duct (38).

**12.** An apparatus (30) for forming a dispersion (10) comprising drops (12), comprising:

- a circulation duct (38) containing drops (12) of a first phase (14) in a second phase (16) that is substantially immiscible with the first phase (14), each drop (12) comprising a core (17) formed from a first phase (14) and a shell (18) formed from a layer of coacervate inserted between the first phase (14) and the second phase (16);
- a container (33) for recovering a dispersion (10) comprising drops (12) and the second phase (16);

**characterized in that** the apparatus (30) includes:

- a reservoir (68) containing a solution (62) increasing the viscosity of the second phase (16);
- at least one injection duct (60; 80) of the solution (62) for increasing the viscosity, connected to the reservoir (68), and emerging in the circulation duct (38) or at the outlet of the circulation duct (38), upstream from the container (33).

**13.** The apparatus (30) according to claim 12, **characterized in that** the circulation duct (38) extends along the local circulation axis of the drops (12) and the second phase (16), the injection duct (60; 80) emerging coaxially with the local axis.

**14.** The apparatus (30) according to any one of claims 12 or 13, **characterized in that** it includes a peripheral duct (60)

for injection of at least part of the solution (62) for increasing the viscosity, emerging at the periphery of the circulation duct (38) and/or a central injection duct (80) for at least part of the solution (62) for increasing the viscosity, emerging at the center of the circulation duct (38).

**15.** The apparatus (30) according to any one of claims 12 to 14, **characterized in that** it includes an assembly forming drops (12) in the circulation duct (38), the drop (12) forming assembly advantageously including:

- a duct (34) for providing a first fluid (36) comprising the first phase (14) and a first precursor polymer of the coacervate contained in the first phase (14);
- a duct (37) forming drops (12) of a first fluid (36) in a second fluid (40) intended to form the second phase (16), the second fluid (40) containing or receiving a second precursor polymer of the coacervate.

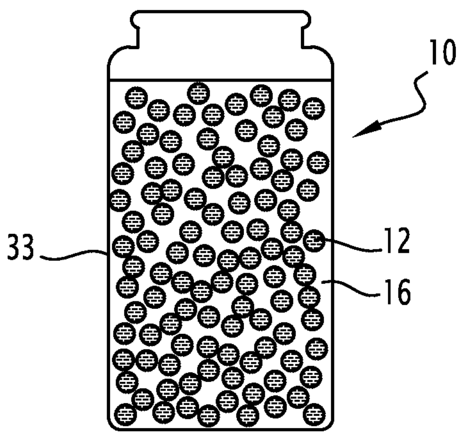


FIG.1

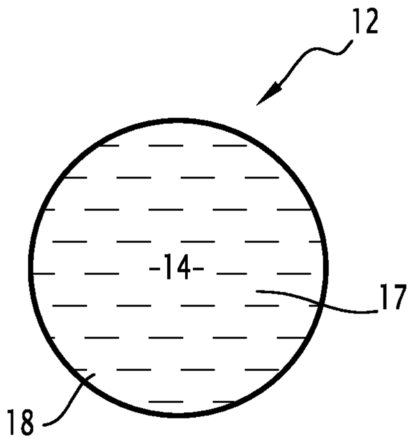


FIG.2

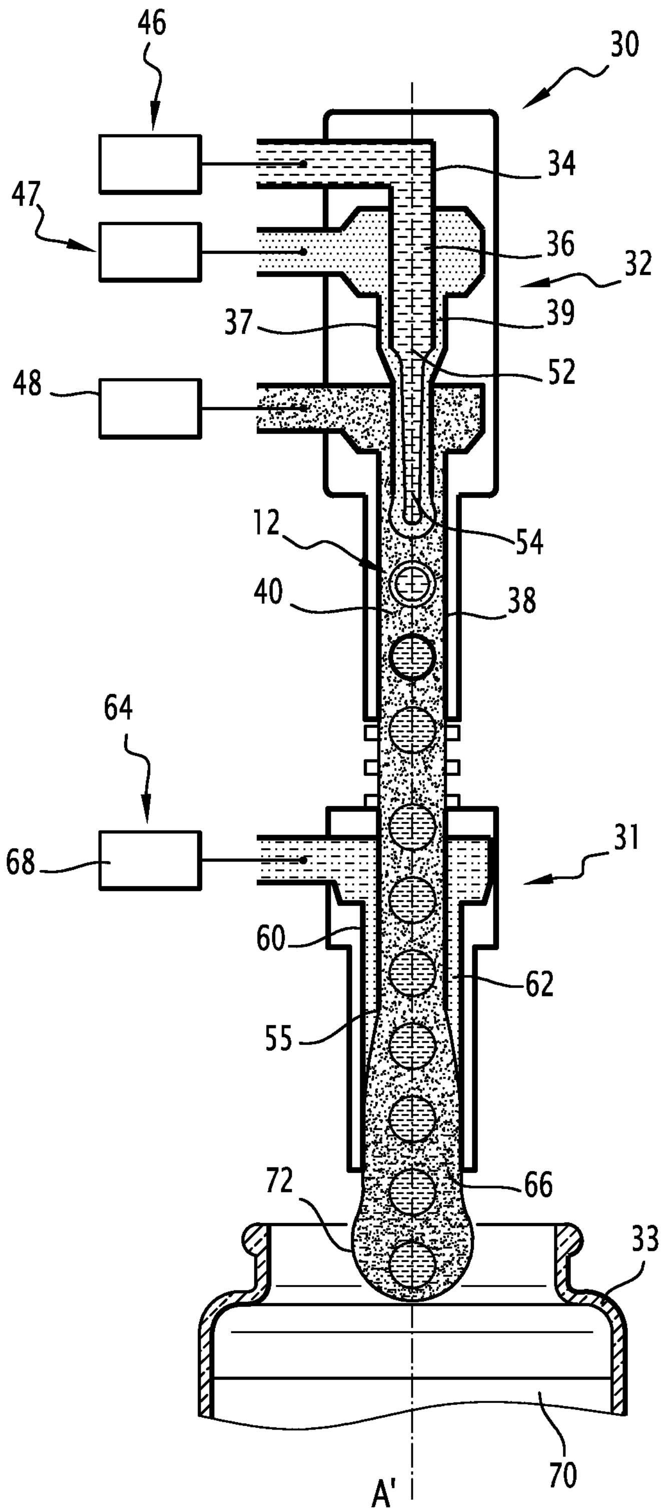


FIG.3

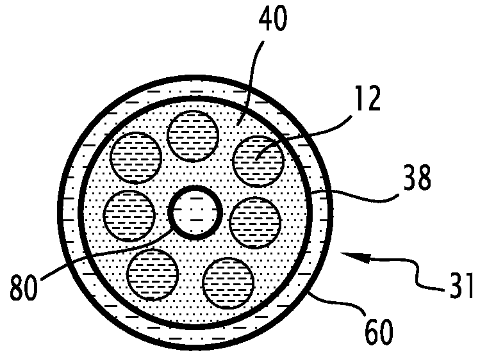


FIG.4

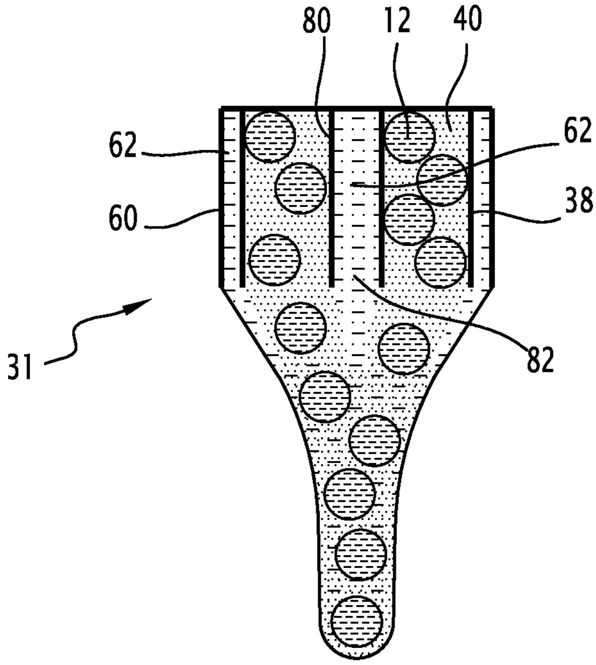


FIG.5

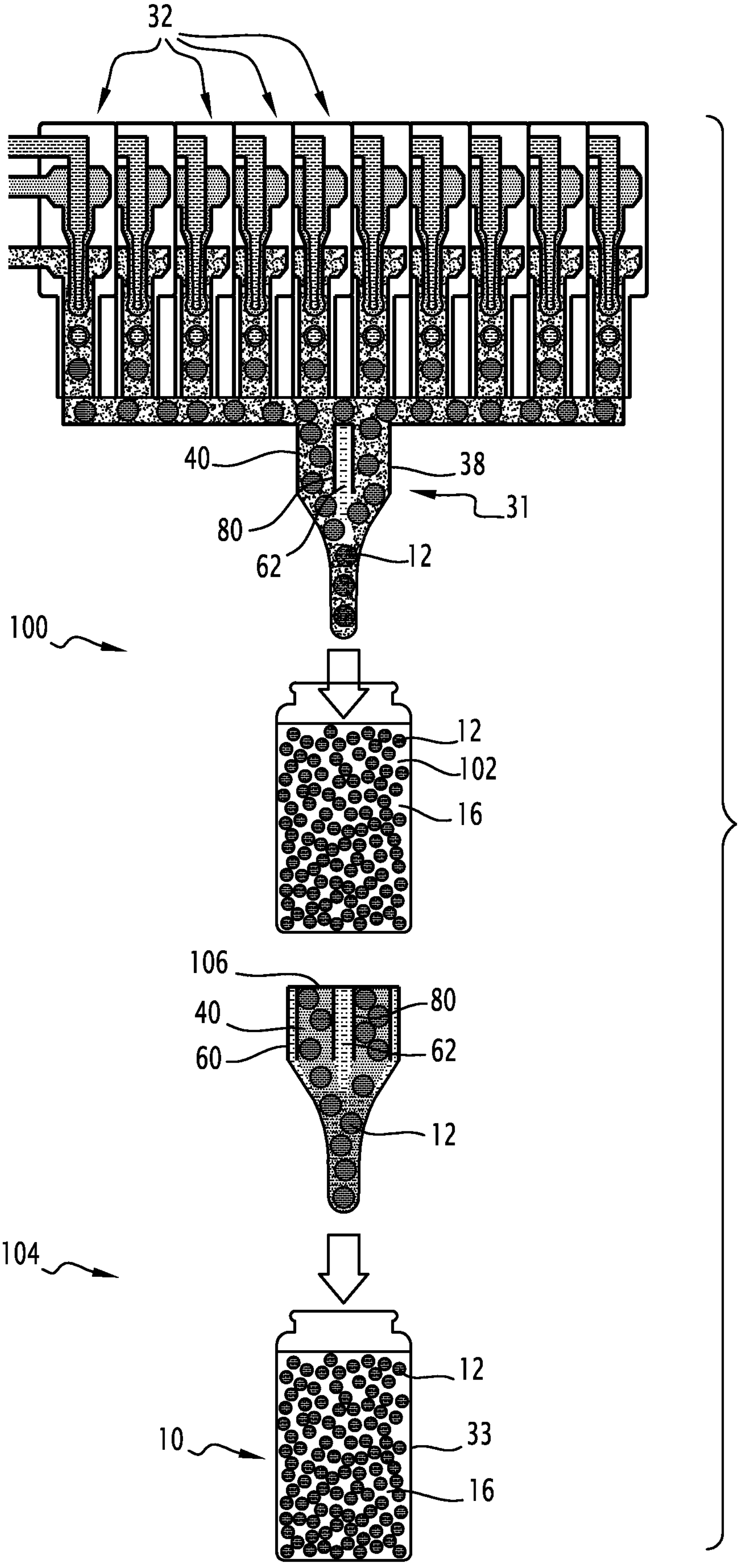


FIG.6

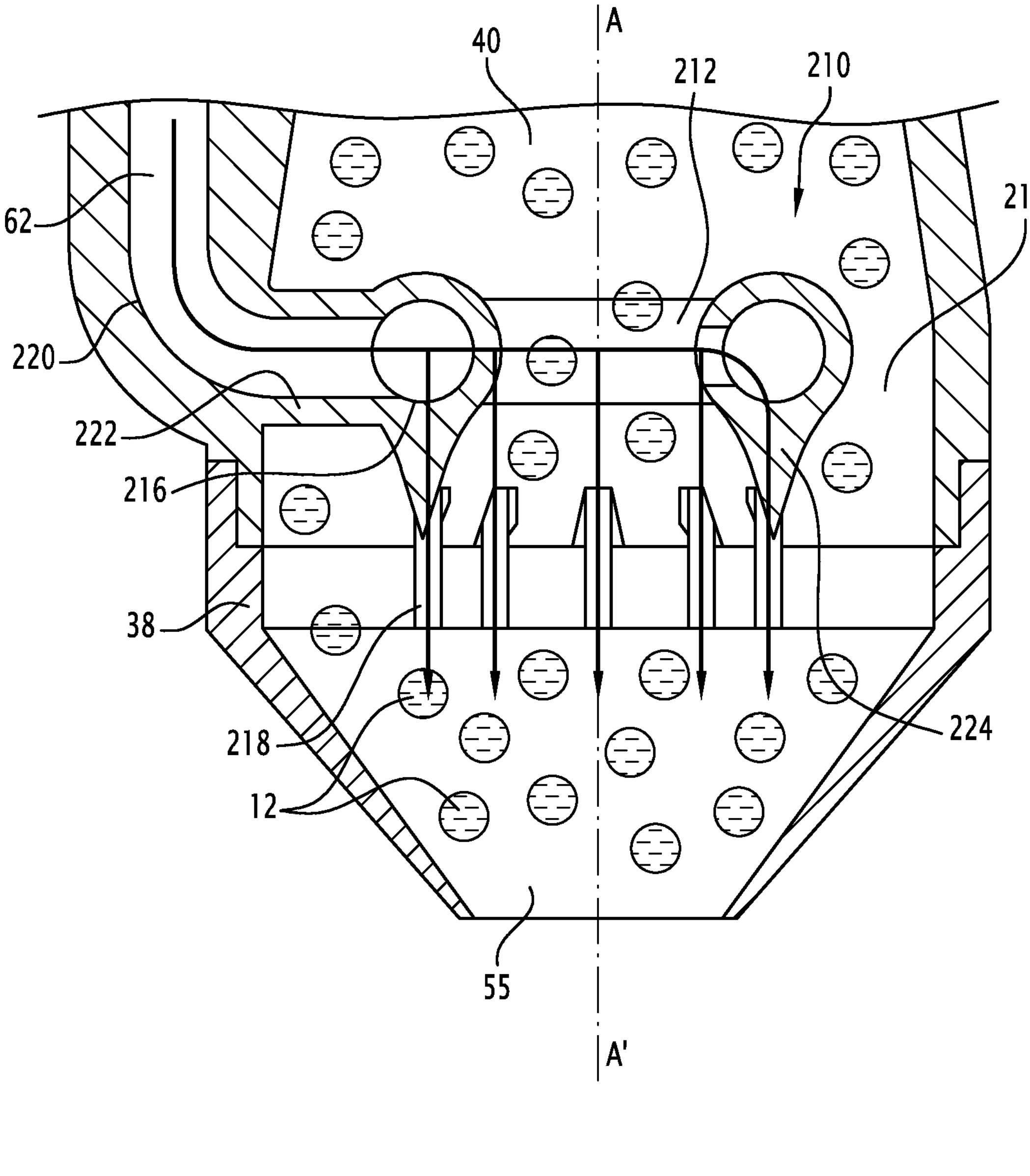


FIG.7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2012120043 A
- FR 2972371 A


### Littérature non-brevet citée dans la description


- **MASON et al.** *J. Coll. Int. Sci.,* 1996, vol. 179, 439-448
- **SATO et al.** *J. Chem. Phys.,* 2007, vol. 111, 1393-1401